# EUROPÄISCHE PATENTSCHRIFT

(11) **EP 0 398 061 B1**
(45) Veröffentlichungstag und Bekanntmachung des Hinweises auf die Patenterteilung: **26.01.1994**
(21) Anmeldenummer: 90108156.2
(22) Anmeldetag: 28.04.1990
(51) Int. Cl.: C07C 57/76, C07C 69/653, C07C 49/163

(54) **Verfahren zur Herstellung von alpha-Fluoracrylsäurederivaten und neue 1,1-Difluor-2-halogenethyl-(halogen) methyl-ketone**
Method of preparing alpha-fluoroacrylic acid derivatives and 1,1-difluoro-2-halogenoethyl(halogeno)methyl-ketones
Procédé pour la fabrication de dérivés de l'acide alpha-fluoroacrylique et 1,1-difluoro-2-halogénoéthyl(halogéno)méthyl cétones

(30) Priorität: 13.05.1989 DE 3915754
(43) Veröffentlichungstag der Anmeldung: 22.11.1990
(73) Patentinhaber: BAYER AG, 51368 Leverkusen (DE)
(72) Erfinder: Gassen, Karl-Rudolf, Dr., D-5068 Odenthal (DE)

(56) Entgegenhaltungen:
- CHEMISCHE BERICHTE, Band 109, Nr. 7, 1976, Seiten 2351-2369, Weinheim/Bergstrasse, DE; M. KAMEL et al.: "Difluorcyclopropan aus Chlordifluormethan und Alkanolat-Ionen in geringer Konzentration"
- PATENT ABSTRACTS OF JAPAN, Band 10, Nr. 258 (C-370)(2314), 4. September 1986; & JP - A - 6185345
- PATENT ABSTRACTS OF JAPAN, Band 8, Nr. 106 (C223)(1543), 18. Mai 1984; & JP - A - 5920242
- PATENT ABSTRACTS OF JAPAN, Band 12, Nr. 37 (C-473)(2884), 4. Februar 1988; & JP - A - 62185039
- PATENT ABSTRACTS OF JAPAN, Band 9, Nr. 324 (C-320)(2047), 19. Dezember 1985; & JP - A - 60158137

## Beschreibung

Die vorliegende Erfindung betrifft ein vorteilhaftes Verfahren zur Herstellung von α-Fluoracrylsäurederivaten und 1,1-Difluor-2-halogenethyl-(halogen)methyl-ketone, die bei diesem Verfahren als Zwischenprodukte erhältlich sind.

Derivate der α-Fluoracrylsäure sind vielseitige organische Zwischenprodukte und können insbesondere als Monomere zur Herstellung von Polymeren für optische Gläser, Lichtwellenleiter und deren Umhüllungen verwendet werden (siehe z.B. EP-A 0 128 517). Die bisher bekannten Verfahren zur Herstellung von α-Fluoracrylsäurederivaten sind nachteilig, weil sie viele Reaktionsstufen erfordern, schlechte Ausbeuten erbringen, schwierig zugängliche und zum Teil toxische Chemikalien benötigten, deren Handhabung besondere sicherheitstechnische Aufwendungen erfordert, nicht reproduzierbar sind und/oder nur die Herstellung kleiner Mengen gestatten.

So ist es bisher bekannt, 2-Fluoracrylsäurefluorid aus Tetrafluoroxetan herzustellen (siehe EP-OS 0 148 490), welches nur mit großem technischem Aufwand zugänglich und sehr toxisch ist. Die Synthese, ausgehend von Fluoressigsäure, hat die gleichen Nachteile (siehe US-PS 3 075 002). Viele Reaktionsschritte benötigt ein Verfahren, das von 1,2-Dibrom-3-chlorpropan ausgeht (siehe Zh. Org. Khim 28, 1173 (1987)). Eine elektrochemische Herstellungsmethode (siehe DE-A 3 704 915) erfordert großen apparativen Aufwand und liefert nicht immer reproduzierbare Resultate.

Es besteht deshalb das Bedürfnis nach einem einfach durchzuführenden und effektiven Verfahren zur Herstellung von α-Fluoracrylsäurederivaten in technischen Mengen.

Es wurde nun ein Verfahren zur Herstellung von α-Fluoracrylsäurederivaten der Formel (I) gefunden 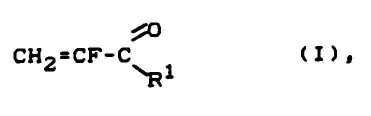
in der
- R¹: für ein Fluoratom, ein Chloratom oder für OR² steht, mit R² = gegebenenfalls substituiertm C₁-bis C₂₀-Alkyl, gegebenenfalls substituiertem C₃-bis C₂₀-Cycloalkyl, gegebenenfalls substituiertem C₆- bis C₂₀-Aryl oder gegebenenfalls substituiertem C₇- bis C₂₀-Aralkyl,
das dadurch gekennzeichnet ist, daß man eine 2,2-Difluor-1-methyl-cyclopropylverbindung der Formel (II) 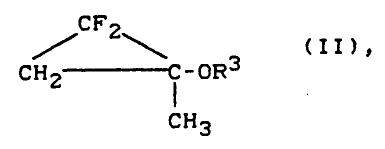
in der
- R³: für C₁- bis C₆-Alkyl oder C₂- bis C₆-Acyl steht,
mit mindestens einem Äquivalent Halogen aus der Gruppe Chlor, Brom und Jod umsetzt und so Verbindungen der Formel (III) erhält

HalCH₂-CF₂-CO-CH₃₋ₙHalₙ (III),

in der
- n: für 0, 1, 2 oder 3 und
- Hal: jeweils unabhängig voneinander für Chlor, Brom oder Jod steht,
diese mit weiterem Halogen in Gegenwart von wäßrigem Alkali zur entsprechenden Carbonsäure der Formel (IV) umsetzt

HalCH₂-CF₂-COOH (IV),

in der
- Hal: für Chlor, Brom oder Jod steht,
diese auf an sich bekannte Weise in das entsprechende Säurechlorid der Formel (V) überführt

HalCH₂-CF₂-COCl (V),

in der
- Hal: für Chlor, Brom oder Jod steht,
zur Herstellung von Verbindungen der Formel (I) mit R¹ = OR² das Säurechlorid der Formel (V) mit einem Alkohol der Formel HOR² zu einem Ester der Formel (VI) verestert

HalCH₂-CF₂-COOR² (VI),

in der
- Hal: für Chlor, Brom oder Jod steht und
- R²: die bei Formel (I) angegebene Bedeutung hat,
und das Säurechlorid der Formel (V) oder den Ester der Formel (VI) partiell dehalogeniert.

Soweit bei den α-Fluoracrylsäurederivaten der Formel (I) R² für einen gegebenenfalls substituierten Alkyl-, Cycloalkyl-, Aryl- oder Aralkylrest steht, kommen als Substituenten beispielsweise Halogenatome, C₁- bis C₆-Alkoxyreste, C₆- bis C₁₀-Aryloxyreste, C₇- bis C₁₂-Aralkoxyreste und/oder 5- oder 6-gliedrige heterocyclische Reste wie Pyridyl, Pyrimidyl und Imidazoyl in Frage. Bevorzugt sind unsubstituierte und mit Halogenatomen, insbesondere mit Fluor und/oder Chlor substituierte Reste der genannten Art. Vorzugsweise steht in Formel (I) R¹ für ein Fluoratom oder für OR², wobei R², gegebenenfalls mit Fluor und/oder Chlor substituiertes C₁-bis C₆-Alkyl, gegebenenfalls mit Fluor und/oder Chlor substituiertes C₃-bis C₆-Cycloalkyl, gegebenfalls mit Fluor und/oder Chlor substituiertes C₆-bis C₁₀-Aryl oder gegebenenfalls mit Fluor und/oder Chlor substituiertes C₇- bis C₁₀-Aralkyl bedeutet. Als Substituent ist Fluor besonders bevorzugt. Die Anzahl der Substituenten kann weit variieren. Bevorzugt sind höher substituierte Reste, insbesondere solche mit einem Fluorierungsgrad von 50 bis 100 %.

Die für das erfindungsgemäße Verfahren als Ausgangsverbindungen benötigten 2,2-Difluor-1-methyl-cyclopropylverbindungen der Formel (II) sind bekannt und gut zugänglich (siehe Chem. Ber. 109, 2351 (1976) und Chem. Pharm. Bull. 27, 3123 (1979)). Vorzugsweise setzt man Verbindungen der Formel (II) ein, bei denen R³ für Methyl, Ethyl oder Acetyl steht, besonders bevorzugt solche mit R³ = Methyl.

Für die Umsetzung zu Verbindungen der Formel (III) wird mindestens ein Äquivalent Halogen aus der Gruppe Chlor, Brom und Jod, vorzugsweise Brom, eingesetzt. Das gewährleistet eine weitgehende bis vollständige Aufspaltung des Cyclopropylringes. Mit ungefähr einem Äquivalent Halogen, z.B. 1,0 bis 1,2 Äquivalenten, erhält man überwiegend die Verbindung der Formel (III) mit n = 0. Werden größere Mengen Halogen eingesetzt, so erhält man überwiegend Verbindungen der Formel (III) mit n = 1, 2 oder 3. Aus wirtschaftlichen Erwägungen ist es im allgemeinen vorteilhaft, nicht mehr als 6 Äquivalente Halogen einzusetzen.

Die Reaktion wird im allgemeinen in Gegenwart eines Verdünnungsmittels durchgeführt, beispielsweise in Gegenwart eines organischen Lösungsmittels oder Wasser. Bevorzugt ist Wasser, wobei man zweckmäßigerweise für eine gute Durchmischung des Reaktionsgemisches Sorge trägt. Die Reaktionstemperatur ist nicht kritisch und kann beispielsweise bei 0 bis 50°C liegen. Vorzugsweise arbeitet man bei 18 bis 25°C.

Es ist nicht erforderlich, die so hergestellte Verbindung der Formel (III) oder das so hergestellte Gemisch von Verbindungen der Formel (III) zu isolieren, insbesondere nicht, wenn man Wasser als Verdünnungsmittel eingesetzt hat.

Den nächsten Reaktionsschritt, die Überführung der Verbindung(en) der Formel (III) in die Carbonsäure der Formel (IV), kann man im gleichen Gefäß wie die vorstehend beschriebene Umsetzung mit Halogen durchführen. Hierzu muß nur mindestens so viel Halogen vorhanden sein, gegebenenfalls durch erneutes Hinzufügen, wie theoretisch für die quantitative Bildung der Verbindung der Formel (III) mit n = 3 sowie zu deren Spaltung zur Carbonsäure der Formel (IV) und dem jeweiligen Haloform benötigt wird. Für die Halogenierung zu(r) Verbindung(en) der Formel (III) und deren Spaltung zur Carbonsäure der Formel (IV) und Haloform werden theoretisch insgesamt 4 Äquivalente Halogen benötigt. Man setzt deshalb insgesamt z.B. 3,8 bis 6, vorzugsweise 4,0 bis 5,5 Äquivalente Halogen ein, wobei man z.B. 1 bis 3,2 Äquivalente in die Umsetzung mit der Cyclopropylverbindung der Formel (II) und z.B. 2,8 bis 5 Äquivalente in die Umsetzung zur Herstellung der Carbonsäure der Formel (IV) einsetzen kann. Man kann auch das insgesamt benötigte Halogen oder überschüssiges Halogen schon zur Umsetzung der Cyclopropylverbindung der Formel (II) einsetzen. Wenn man zwei- oder mehrmals Halogen hinzufügt, kann das Halogen jeweils gleich oder verschieden sein, z.B. nur Brom, aber auch z.B. zunächst Brom und dann Chlor. Chlor kann man z.B. in elementarer Form oder in Form einer Hypochloritlösung umsetzen.

Die Überführung der Verbindung(en) der Formel (III) in die Carbonsäure der Formel (IV) kann man beispielsweise bei 0 bis 50°C vornehmen. Vorzugsweise arbeit man bei 18 bis 25°C. Das dabei als zweites Produkt entstehende Haloform kann aus dem Reaktionsgemisch in reiner Form gewonnen werden, z.B. durch Destillation, und ist dann in bekannter Weise verwendbar, z.B., wenn es sich um Bromoform handelt, zur Trennung von Mineralgemischen oder als Eichflüssigkeit.

Das für die Umsetzung der Verbindung(en) der Formel (III) zu der Carbonsäure der Formel (IV) benötigte wäßrig-alkalische Milieu kann beispielsweise durch Zusatz von wäßrigem Alkali, beispielsweise wäßriger Natron- oder Kalilauge, hergestellt werden. Man kann beispielsweise so viel Alkali zusetzen, daß während und nach der Reaktion der pH-Wert im Bereich von 8 bis 14 liegt.

Die Carbonsäure der Formel (IV) kann man beispielsweise aus dem Reaktionsgemisch isolieren, indem man das Reaktionsgemisch ansäuert, z.B. mit Mineralsäure, und die organische Phase abtrennt und destilliert.

Die Überführung der Carbonsäure der Formel (IV) in das entsprechende Carbonsäurechlorid der Formel (V) kann auf an sich bekannte Weise durchgeführt werden, beispielsweise durch Umsetzung mit Phosphortrichlorid, Phosphorpentachlorid, Sulfurylchlorid oder Thionylchlorid. Vorzugsweise verwendet man einen kleinen Überschuß an Thionylchlorid bei Temperaturen im Bereich von 15 bis 80°C. Das Säurechlorid der Formel (V) kann gegebenenfalls durch direkte Destillation isoliert werden.

Wenn man α-Fluoracrylsäurederivate der Formel (I) mit R¹ = OR² herstellen mochte, schließt sich an die Herstellung des Carbonsäurechlorids der Formel (V) dessen Veresterung mit einem Alkohol der Formel R²OH an. Der Alkohol wird dabei zweckmäßigerweise so gewählt, daß sein R²O-Rest dem Rest R¹ in der Verbindung der Formel (I) entspricht, die man herstellen möchte. So können spätere Umesterungsreaktionen entfallen. Alkohole der Formel R²OH sind gut zugänglich, insbesondere diejenigen Alkohole der Formel R²OH, bei denen R² die bei Formel (I) als bevorzugt angegebenen Bedeutungen hat. Die Veresterung kann auf an sich bekannte Weise erfolgen, beispielsweise, indem man das Säurechlorid und den Alkohol, gegebenenfalls in Gegenwart eines Verdünnungsmittels, erwärmt. Nach einer üblichen Aufarbeitung kann man so einen Ester der Formel (VI) erhalten.

Einige Ester der Formel (VI), z.B. mit Hal = Brom und R² = Ethyl, sind bekannte. Sie wurden durch Fluorierung von 3-Brombrenztraubensäureester mit Schwefeltetrafluorid hergestellt (siehe J. Med. Chem. 31, 370 (1988)). Wegen des großen sicherheitstechnischen Aufwands für die Handhabung von Schwefeltetrafluorid ist dieses Verfahren allenfalls im Labormaßstab anwendbar und somit dem erfindungsgemäßen Verfahren weit unterlegen.

Zur Herstellung von α-Fluoracrylsäurederivaten der Formel (I) mit R¹ = OR² ist der Ester der Formel (VI), oder zur Herstellung von α-Fluoracrylsäurederivaten der Formel (I) mit R¹ = Fluor oder Chlor das Carbonsäurechlorid der Formel (V) partiell zu dehalogenieren. Diese partielle Dehalogenierung kann elektrochemisch oder mit chemischen Dehalogenierungsmitteln durchgeführt werden. Chemische Dehalogenierungsmittel sind beispielsweise Metallhalogenide, in denen das Metall in einer niedrigen Oxidationsstufe vorliegt, z.B. CrCl₂, und Metalle, z.B. Magnesium, Eisen oder Zink. Vorzugsweise verwendet man Zink, das gegebenenfalls aktiviert sein kann (siehe L.F. Fieser und M. Fieser, Reagents for Org. Synth., Wiley, Vol 1, S. 1276 (1967)). Üblicherweise arbeitet man in Gegenwart eines Verdünnungsmittel. In Frage kommen beispielsweise verschiedene Glyme, Tetrahydrofuran, Dioxan, Wasser und beliebige Gemische davon. Geeignete Temperaturen sind beispielsweise solche von 20 bis 250°C, insbesondere solche von 50 bis 200°C.

Eine bevorzugte Ausführungsform der partiellen Dehalogenierung besteht darin, daß man das Carbonsäurechlorid der Formel (V) in Gegenwart von Diglyme mit Zink bei 80 bis 110°C umsetzt und das dabei gebildete α-Fluoracrylsäurederivat der Formel (I) mit R¹ = Fluor oder Chlor abdestilliert. Überraschend ist dabei, daß während der partiellen Dehalogenierung ein Halogenaustausch stattfinden kann, insbesondere wenn man durch langsameres Zutropfen des Carbonsäurechlorids der Formel (V) und langsameres Abdestillieren der Reaktionsprodukte (steuerbar durch Temperatur und/oder Druck) eine längere Verweilzeit realisiert. So erhält man aus einem Carbonsäurechlorid der Formel (V) vorzugsweise das α-Fluoracrylsäurefluorid (Formel (I), R¹ = F). Bei schnellerem Zutropfen des Carbonsäurechlorids der Formel (V) und schnellerem Abdestillieren der Reaktionsprodukte erhält man vorzugsweise α-Fluoracrylsäurechlorid (Formel (I), R¹ = Cl). Soweit Gemische von α-Fluoracrylsäurefluorid und -chlorid anfallen, kann man diese durch Destillation trennen.

Eine weitere besondere Ausführungsform der partiellen Dehalogenierung besteht darin, daß man einen Ester der Formel (VI) in Gegenwart von Wasser mit Zink bei 50 bis 100°C umsetzt und aus dem Reaktionsgemisch Wasser und das gebildete α-Fluoracrylsäurederivat der Formel (I) mit R¹ = OR² abdestilliert. Aus dem Destillat kann man nach üblichen Methoden das α-Fluoracrylsäurederivat in reiner Form gewinnen, z.B. durch Phasentrennung und Trocknung und Destillation der organischen Phase. Es ist vorteilhaft, während der Reaktion durch Zusatz einer Säure, z.B. verdünnter Schwefelsäure, den pH-Wert des Reaktionsgemisches zwischen 7 und 2 zu halten.

Da die α-Fluoracrylsäurederivate der Formel (I) häufig polymerisationsempfindlich sind, empfiehlt es sich im allgemeinen, bei deren Herstellung und Lagerung übliche Polymerisationsinhibitoren hinzuzufügen, z.B. 4-Methoxyphenol.

Die vorliegende Erfindung gestattet die Herstellung von α-Fluoracrylsäurederivaten auf einfache Weise, in wenigen Reaktionsstufen (die Überführung des Ausgangsmaterials der Formel (II) zu der Carbonsäure der Formel (IV) kann als Eintopftreaktion durchgeführt werden), mit guten Ausbeuten, unter Verwendung wenig- oder nicht-toxischer Chemikalien und in üblichen Apparaturen. Sie eignet sich deshalb besonders für die Herstellung von α-Fluoracrylsäurederivaten in technischen Mengen.

Die vorliegende Erfindung betrifft weiterhin neue 1,1-Difluor-2-halogenethyl-(halogen)methyl-ketone der Formel (IIIa)

Hal′CH₂-CF₂-CO-CH₃₋ₙHalₙ (IIIa),

in der
- n: für 0, 1, 2 oder 3 steht,
- Hal: für Chlor, Brom oder Jod steht und
im Falle n = 1, 2 oder 3 Hal′ für Chlor, Brom oder Jod und
im Falle n = 0 Hal′ für Brom steht.

Es handelt sich hierbei beispielsweise um
1,1-Difluor-2-bromethyl-methyl-keton,
1,1-Difluor-2-bromethyl-halogenmethyl-ketone,
1,1-Difluor-2-bromethyl-dihalogenmethyl-ketone,
1,1-Difluor-2-bromethyl-trihalogenmethyl-ketone,
1,1-Difluor-2-chlorethyl-halogenmethyl-ketone,
1,1-Difluor-2-chlorethyl-dihalogenmethyl-ketone,
1,1-Difluor-2-chlorethyl-trihalogenmethyl-ketone,
1,1-Difluor-2-jodethyl-halogenmethyl-ketone,
1,1-Difluor-2-jodethyl-dihalogenmethyl-ketone und
1,1-Difluor-2-jodethyl-trihalogenmethyl-ketone,
wobei Halogen jeweils Chlor, Brom oder Jod bedeutet.

Bevorzugte Verbindungen der Formel (IIIa) sind solche, bei denen n für 0, 1 oder 2, Hal für Chlor oder Brom und Hal′ für Brom steht, also z.B.
1,1-Difluor-2-bromethyl-brommethyl-keton,
1,1-Difluor-2-bromethyl-dibrommethyl-keton und
1,1-Difluor-2-bromethyl-methyl-keton.

Die Herstellung von Verbindungen der Formel (IIIa) kann erfolgen wie oben beschrieben. Zu beachten ist dabei, daß man mit der Menge Halogen, die man auf die 2,2-Difluor-1-methyl-cyclopropylverbindung der Formel (I) einwirken läßt, steuern kann, ob überwiegend 1,1-Difluor-2-halogenethyl-(halogen)methyl-ketone der Formel (III) mit n = 0, 1, 2 oder 3 gebildet werden. Setzt man beispielsweise 1,0 bis 1,2 Äquivalente Halogen ein, so entsteht überwiegend die Verbindung der Formel (IIIa) mit n = 0. Entsprechend entstehen überwiegend Verbindungen der Formel (IIIa) mit n = 1, 2 oder 3, wenn man beispielsweise 1,8 bis 2,2 bzw. 2,8 bis 3,2 bzw. 3,8 bis 4,2 Äquivalente Halogen einsetzt. Die Verbindungen der Formel (IIIa) kann man aus dem Reaktionsgemisch isolieren, indem man beispielsweise die organische Phase abtrennt und gegebenenfalls nach einer Trocknung destilliert. Es kann vorteilhaft sein, eventuell noch vorhandenes überschüssiges Halogen vor der Destillation zu entfernen, beispielsweise durch Schütteln mit einer Hydrogensulfit-Lösung.

Die Verbindungen der Formel (III) können, wie oben beschrieben, als Zwischenprodukte zur Herstellung von α-Fluoracrylsäurederivaten verwendet werden, die ihrerseits Monomere zur Herstellung von Polymeren sind, die beispielsweise für optische Gläser, Lichtwellenleiter und deren Umhüllungen Verwendung finden können.

### Beispiel 1

45 g (0,37 Mol) 2,2-Difluor-1-methylcyclopropyl-methylether, 240 g (1,5 Mol) Brom und 300 ml Wasser wurden 18 Stunden bei Raumtemperatur gerührt. Danach wurde das Gemisch mit Diethylether extrahiert, die organische Phase getrocknet und 85,5 g (67 % der Theorie) eines Produktes der Formel (III) mit n = 2 und Hal = Brom isoliert, welches bei 20 mbar bei 101 bis 103°C siedete, im IR-Spektrum die C=O-Absorption bei 1770 cm⁻¹ aufwies, ¹H-NMR-Absorptionen bei 3,79 und 6,40 ppm zeigte, sowie im MS-Spektrum das Isotopenmuster des Molekülions mit 3 Bromatomen bei m/e 342, 344 und 346 erzeugte.

### Beispiel 2

Es wurde verfahren wie in Beispiel 1, jedoch wurden nur 0,5 Mol Brom eingesetzt und das Produkt der Formel (III) mit n = 1 und Hal = Brom erhalten, welches im MS-Spektrum das Isotopenmuster des Molekülions mit 2 Bromatomen bei m/e 264 und 266 erzeugte.

### Beispiel 3

71 g (1 Mol) Chlor wurden in eine Mischung aus 122 g (1 Mol) 2,2-Difluor-1-methylcyclopropyl-methylether und 700 ml Wasser geleitet und sonst verfahren wie bei Beispiel 1. Es wurden 37 g (26 % der Theorie) des Produktes der Formel (III) mit n = 0 und Hal = Chlor erhalten, welches bei 104 bis 105°C (bei Normaldruck) siedete, im IR-Spektrum die C=O-Absorption bei 1740 cm⁻¹ aufwies und ¹H-NMR-Absorptionen bei 2,41 und 3,87 ppm zeigte.

### Beispiel 4

Mit einer Mischung aus 12 g (0,1 Mol) 2,2-Difluor-1-methylcyclopropyl-methylether, 70 ml Wasser und 40 g (0,16 Mol) Jod wurde analog der Arbeitsweise von Beispiel 1 verfahren. Es wurden 2,5 g (11 % der Theorie) des Produkts der Formel (III) mit n = 0 und Hal = Jod erhalten, welches im IR-Spektrum die C=O-Absorption bei 1745 cm⁻¹ aufwies und im Massenspektrum unter anderem bei m/e = 234, 191/190, 127, 64 und 43 charakteristische Signale lieferte.

### Beispiel 5

45 g (0,37 Mol) 2,2-Difluor-1-methylcyclopropyl-methylether wurden mit 300 g (1,88 Mol) Brom und 300 ml Wasser 18 Stunden bei Raumtemperatur gerührt. Dann wurden unter Eiskühlung 10 Mol-Äquivalente 45 %ige Natronlauge zugetropft und weitere 15 Stunden gerührt. Dann wurde das Gemisch durch Schütteln mit Natriumhydrogensulfit-Lösung entfernt, mit konzentrierter Salzsäure angesäuert und mit Diethylether extrahiert. Nach dem Trocknen der organischen Phase wurden durch Destillation 44,8 g (64 % der Theorie) reine Säure der Formel (IV) mit Hal = Brom erhalten, die bei 18 mbar. bei 90°C siedete, einen Schmelzpunkt von 49 bis 50°C aufwies, im IR-Spektrum die C=O-Absorption bei 1760 cm⁻¹ zeigte und im ¹H-NMR-Spektrum bei 3,78 und 10,28 ppm Resonanzen ergab.

### Beispiel 6

Es wurde verfahren wie in Beispiel 5, jedoch wurde als Ausgangsprodukt die Verbindung der Formel (III) mit n = 2 und Hal = Brom (Herstellung siehe Beispiel 1) eingesetzt und 2,5 Äquivalente Brom. Auf diese Weise wurde ebenfalls die Verbindung der Formel (IV) mit Hal = Brom erhalten, diesmal jedoch in einer Ausbeute von 79 % der Theorie.

### Beispiel 7

50 g (0,26 Mol) 3-Brom-2,2-difluor-propionsäure und 37,5 g (0,32 Mol) Thionylchlorid wurden bei Raumtemperatur bis zum Ende der Gasentwicklung gerührt und anschließend direkt destilliert. Es wurden 46 g (84 % der Theorie) der Verbindung der Formel (V) mit Hal = Brom erhalten, die einen Siedepunkt von 111 bis 112°C (bei Normaldruck) aufwies, die im IR-Spektrum die C=O-Absorption bei 1790 cm⁻¹ zeigte und im ¹H-NMR-Spektrum ein Triplett bei 3,79 ppm hervorrief.

### Beispiel 8

19 g (0,091 Mol) 3-Brom-2,2-difluorpropionsäurechlorid und 60 ml Ethanol wurden 3 Stunden zum Rückfluß erhitzt. Dann wurde mit Wasser verdünnt, mit Dichlormethan extrahiert, entsäuert und die organischen Phasen getrocknet und destilliert. So wurden 14,5 g (73 % der Theorie) einer Verbindung der Formel (VI) mit Hal = Brom und R² = Ethyl erhalten, welche bei 65 mbar bei 78 bis 80°C siedete und im IR-Spektrum die C=O-Absorption bei 1760 cm⁻¹ aufwies.

### Beispiel 9

53 g (0,25 Mol) 3-Brom-2,2-difluor-propionsäurechlorid wurden zu einer auf 100°C erwärmten Suspension aus 25 g (0,38 Mol) Zink und 50 ml Diglyme getropft. Während des Zutropfens destillierte bereits ein Teil des Produktes ab. Zur Vervollständigung des Umsatzes wurde die Badtemperatur auf 170°C erhöht und so weiteres Produkt abdestilliert. Es wurden insgesamt 15 g (65 % der Theorie) der Verbindung der Formel (I) mit R¹ = Fluor erhalten, welche bei 29 bis 31°C (bei Normaldruck) siedete und im IR-Spektrum die C=O-Absorption bei 1830 cm⁻¹ aufwies.

### Beispiel 10

Beispiel 9 wurde wiederholt, wobei das Produkt durch eine weniger gut wirksame Kolonne abdestillierte. In diesem Fall enthielt das Produkt eine kleine Menge α-Fluoracrylsäurechlorid.

### Beispiel 11

5 g (0,023 Mol) 3-Brom-2,2-difluorpropionsäureethylester wurden in eine auf 100°C erwärmte Suspension aus 5 g (0,077 Mol) Zink, 10 ml Wasser und 250 mg konzentrierte Schwefelsäure getropft. Das Produkt destillierte dabei als Azeotrop in eine Vorlage ab, die 10 mg 4-Methoxyphenol enthielt. Durch Phasentrennung und Trocknung wurden 1,5 g (55 % der Theorie) reine Verbindung der Formel (I) mit R¹ = OR² mit R² = Ethyl erhalten, welche im ¹H-NMR-Spektrum Absorptionen bei 1,36, 4,38, 5,31 und 5,68 ppm aufwies.

## Patentansprüche

1. Verfahren zur Herstellung von α-Fluoracrylsäurederivaten der Formel (I) 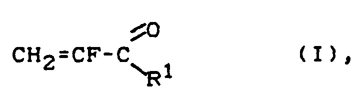 in der
R¹ für ein Fluoratom, ein Chloratom oder für OR² steht, mit R² = gegebenenfalls substituiertem C₁-bis C₂₀-Alkyl, gegebenenfalls substituiertem C₃-bis C₂₀-Cycloalkyl, gegebenenfalls substituiertem C₆- bis C₂₀-Aryl oder gegebenenfalls substituiertem C₇- bis C₂₀-Aralkyl,
dadurch gekennzeichnet, daß man eine 2,2-Difluor-1-methyl-cyclopropylverbindung der Formel (II) 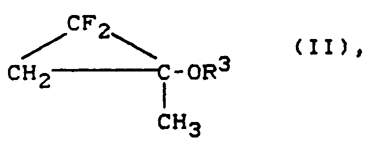 in der
R³ für C₁- bis C₆-Alkyl oder C₂- bis C₆-Acyl steht,
mit mindestens einem Äquivalent Halogen aus der Gruppe Chlor, Brom und Jod umsetzt und so Verbindungen der Formel (III) erhält
HalCH₂-CF₂-CO-CH₃₋ₙHalₙ (III),
in der
n für 0, 1, 2 oder 3 und
Hal jeweils unabhängig voneinander für Chlor, Brom oder Jod steht,
diese mit weiterem Halogen in Gegenwart von wäßrigem Alkali zur entsprechenden Carbonsäure der Formel (IV) umsetzt
HalCH₂-CF₂-COOH (IV),
in der
Hal für Chlor, Brom oder Jod steht,
diese auf an sich bekannte Weise in das entsprechende Säurechlorid der Formel (V) überführt
HalCH₂-CF₂-COCl (V),
in der
Hal für Chlor, Brom oder Jod steht,
zur Herstellung von Verbindungen der Formel (I) mit R¹ = OR² das Säurechlorid der Formel (V) mit einem Alkohol der Formel HOR² zu einem Ester der Formel (VI) verestert
HalCH₂-CF₂-COOR² (VI),
in der
Hal für Chlor, Brom oder Jod steht und
R² die bei Formel (I) angegebene Bedeutung hat,
und das Säurechlorid der Formel (V) oder den Ester der Formel (VI) partiell dehalogeniert.

2. Verfahren nach Anspruch 1, dadurch gekennzeichnet, daß man als Halogen Brom verwendet.

3. Verfahren nach Ansprüchen 1 und 2, dadurch gekennzeichnet, daß man als Dehalogenierungsmittel Zink verwendet.

4. Verfahren nach Ansprüchen 1 bis 3, dadurch gekennzeichnet, daß man Verbindungen der Formel (I), in denen R¹ für ein Fluoratom oder ein Chloratom steht herstellt, indem man eine 2,2-Difluor-1-methyl-cyclopropylverbindung der Formel (II) 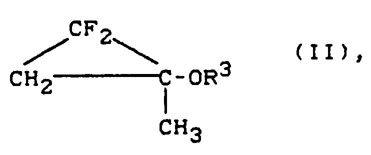 in der
R³ für C₁- bis C₆-Alkyl oder C₂- bis C₆-Acyl steht,
mit mindestens einem Äquivalent Halogen aus der Gruppe Chlor, Brom und Jod umsetzt und so Verbindungen der Formel (III) erhält
HalCH₂-CF₂-CO-CH₃₋ₙHalₙ (III),
in der
n für 0, 1, 2 oder 3 und
Hal jeweils unabhängig voneinander für Chlor, Brom oder Jod steht,
diese mit weiterem Halogen in Gegenwart von wäßrigem Alkali zur entsprechenden Carbonsäure der Formel (IV) umsetzt
HalCH₂-CF₂-COOH (IV),
in der
Hal für Chlor, Brom oder Jod steht,
diese auf an sich bekannte Weise in das entsprechende Säurechlorid der Formel (V) überführt
HalCH₂-CF₂-COCl (V),
in der
Hal für Chlor, Brom oder Jod steht,
und das Säurechlorid der Formel (V) partiell dehalogeniert.

5. Verfahren nach Anspruch 4, dadurch gekennzeichnet, daß man Verbindungen der Formel (I), in der R¹ für ein Fluoratom steht herstellt, indem man das Säurechlorid der Formel (V) langsam in die Dehalogenierung zutropft und die Reaktionsprodukte langsam abdestilliert.

6. 1,1-Difluor-2-halogenethyl-(halogen)methyl-ketone der Formel (IIIa)
Hal′CH₂-CF₂-CO-CH₃₋ₙHalₙ (IIIa),
in der
n für 0, 1, 2 oder 3 steht,
Hal für Chlor, Brom oder Jod steht und
im Falle n = 1, 2 oder 3 Hal′ für Chlor, Brom oder Jod und
im Falle n = 0 Hal′ für Brom steht.

7. 1,1-Difluor-2-halogenethyl-(halogen)methyl-ketone gemäß Anspruch 6, dadurch gekennzeichnet, daß es sich um
1,1-Difluor-2-bromethyl-brommethyl-keton,
1,1-Difluor-2-bromethyl-dibrommethyl-keton oder
1,1-Difluor-2-bromethyl-methyl-keton
handelt.

## Claims

1. Process for the preparation of α-fluoroacrylic acid derivatives of the formula (I) 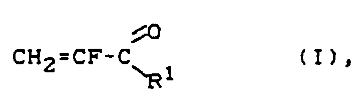 in which
R¹ represents a fluorine atom, a chlorine atom or OR² where R² = optionally substituted C₁- to C₂₀-alkyl, optionally substituted C₃- to C₂₀-cycloalkyl, optionally substituted C₆- to C₂₀-aryl or optionally substituted C₇- to C₂₀-aralkyl,
characterized in that a 2,2-difluoro-1-methyl-cyclopropyl compound of the formula (II) 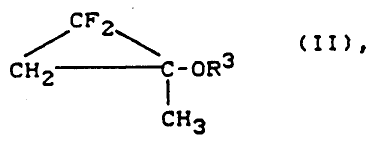 in which
R³ represents C₁- to C₆-alkyl or C₂- to C₆-acyl,
is reacted with at least one equivalent of halogen from the group comprising chlorine, bromine and iodine and compounds are thus obtained of the formula (III)
HalCH₂-CF₂-CO-CH₃₋ₙHalₙ (III),
in which
n represents 0, 1, 2 or 3 and
Hal in each case independently of one another represents chlorine, bromine or iodine,
this is reacted with further halogen in the presence of aqueous alkali to give the corresponding carboxylic acid of the formula (IV)
HalCH₂-CF₂-COOH (IV),
in which
Hal represents chlorine, bromine or iodine,
this is converted in a manner known per se into the corresponding acid chloride of the formula (V)
HalCH₂-CF₂-COCl (V),
in which
Hal represents chlorine, bromine or iodine,
for the preparation of compounds of the formula (I) where R¹ = OR², the acid chloride of the formula (V) is esterified with an alcohol of the formula HOR² to give an ester of the formula (VI)
HalCH₂-CF₂-COOR² (VI),
in which
Hal represents chlorine, bromine or iodine and
R² has the meaning given in formula (I),
and the acid chloride of the formula (V) or the ester of the formula (VI) is partially dehalogenated.

2. Process according to Claim 1, characterized in that bromine is used as the halogen.

3. Process according to Claims 1 and 2, characterized in that zinc is used as the dehalogenating agent.

4. Process according to Claims 1 to 3, characterized in that compounds of the formula (I), in which R¹ represents a fluorine atom or a chlorine atom, are prepared by reacting a 2,2-difluoro-1-methyl-cyclopropyl compound of the formula (II) 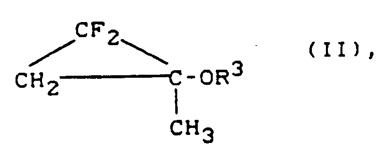 in which
R³ represents C₁- to C₆-alkyl or C₂- to C₆-acyl,
is reacted with at least one equivalent of halogen from the group comprising chlorine, bromine and iodine and compounds are thus obtained of the formula (III)
HalCH₂-CF₂-CO-CH₃₋ₙHalₙ (III),
in which
n represents 0, 1, 2 or 3 and
Hal in each case independently of one another represents chlorine, bromine or iodine,
this is reacted with further halogen in the presence of aqueous alkali to give the corresponding carboxylic acid of the formula (IV)
HalCH₂-CF₂-COOH (IV),
in which
Hal represents chlorine, bromine or iodine,
this is converted in a manner known per se into the corresponding acid chloride of the formula (V)
HalCH₂-CF₂-COCl (V),
in which
Hal represents chlorine, bromine or iodine,
and the acid chloride of the formula (V) is partially dehalogenated.

5. Process according to Claim 4, characterized in that compounds of the formula (I), in which R¹ represents a fluorine atom, are prepared by slowly adding the acid chloride of the formula (V) dropwise to the dehalogenation and slowly removing the reaction products by distillation.

6. 1,1-Difluoro-2-halogenoethyl (halogeno)methyl ketones of the formula (IIIa)
Hal'CH₂-CF₂-CO-CH₃₋ₙHalₙ (IIIa),
in which
n represents 0, 1, 2 or 3,
Hal represents chlorine, bromine or iodine and
if n = 1, 2 or 3, Hal' represents chlorine, bromine or iodine and
if n = 0, Hal' represents bromine.

7. 1,1-Difluoro-2-halogenoethyl (halogeno)methyl ketones according to Claim 6, characterized in that they are
1,1-difluoro-2-bromoethyl bromomethyl ketone,
1,1-difluoro-2-bromoethyl dibromomethyl ketone or
1,1-difluoro-2-bromoethyl methyl ketone.

## Revendications

1. Procédé de préparation de dérivés de l'acide α-fluoroacrylique de formule (I) 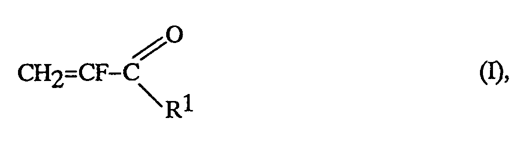 dans laquelle
R¹ représente un atome de fluor, un atome de chlore ou OR² dans lequel R² représente un alkyle en C₁-C₂₀ éventuellement substitué, un cycloalkyle en C₃-C₂₀ éventuellement substitué, un aryle en C₆-C₂₀ éventuellement substitué ou un aralkyle en C₇-C₂₀ éventuellement substitué,
caractérisé en ce que l'on fait réagir un composé 2,2-difluoro-1-méthyl-cyclopropylique de formule (II) 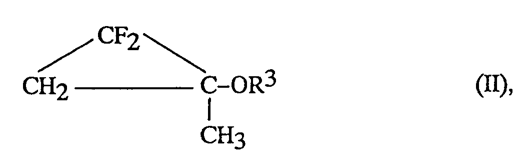 dans laquelle
R³ représente un alkyle en C₁-C₆ ou un acyle en C₂-C₆,
avec au moins un équivalent d'halogène du groupe du chlore, du brome et de l'iode, pour obtenir des composés de formule (III)
HalCH₂-CF₂-CO-CH₃₋ₙHalₙ (III),
dans laquelle
n représente 0, 1, 2 ou 3 et
les symboles Hal représentent indépendamment les uns des autres des atomes de chlore, de brome ou d'iode,
on fait réagir ces composés avec un supplément d'halogène en présence d'alcali aqueux pour former l'acide carboxylique correspondant de formule (IV)
HalCH₂-CF₂-COOH (IV),
dans laquelle Hal représente le chlore, le brome ou l'iode,
on convertit celui-ci de manière connue en soi en le chlorure d'acide correspondant de formule (V)
HalCH₂-CF₂-COCl (V),
dans laquelle
Hal représente le chlore, le brome ou l'iode,
pour la préparation de composés de formule (I) avec R¹ = OR², on estérifie le chlorure d'acide de formule (V) avec un alcool de formule HOR² pour former un ester de formule (VI)
HalCH₂-CF₂-COOR² (VI),
dans laquelle
Hal représente le chlore, le brome ou l'iode, et
R² a la signification indiquée pour la formule (I),
et on déshalogène partiellement le chlorure d'acide de formule (V) ou l'ester de formule (VI).

2. Procédé selon la revendication 1, caractérisé en ce que l'on utilise le brome comme halogène.

3. Procédé selon les revendications 1 et 2, caractérisé en ce que l'on utilise le zinc comme agent de déshalogénation.

4. Procédé selon les revendications 1 à 3, caractérisé en ce que l'on prépare des composés de formule (I) dans lesquels R¹ représente un atome de fluor ou un atome de chlore en faisant réagir un composé 2,2-difluoro-1-méthyl-cyclopropylique de formule (II) 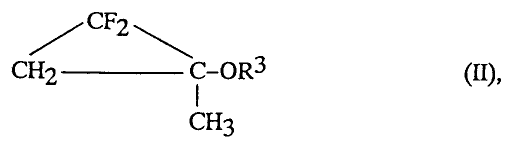 dans laquelle
R³ représente un alkyle en C₁-C₆ ou un acyle en C₂-C₆,
avec au moins un équivalent d'halogène du groupe du chlore, du brome et de l'iode, pour obtenir des composés de formule (III)
HalCH₂-CF₂-CO-CH₃₋ₙHalₙ (III),
dans laquelle
n représente 0, 1, 2 ou 3 et
les symboles Hal représentent indépendamment les uns des autres des atomes de chlore, de brome ou d'iode,
on fait réagir ces composés avec un supplément d'halogène en présence d'alcali aqueux pour former l'acide carboxylique correspondant de formule (IV)
HalCH₂-CF₂-COOH (IV),
dans laquelle Hal représente le chlore, le brome ou l'iode,
on convertit celui-ci de manière connue en soi en le chlorure d'acide correspondant de formule (V)
HalCH₂-CF₂-COCl (V),
dans laquelle
Hal représente le chlore, le brome ou l'iode,
et on déshalogène partiellement le chlorure d'acide de formule (V).

5. Procédé selon la revendication 4, caractérisé en ce que l'on prépare des composés de formule (I) dans laquelle R¹ représente un atome de fluor en ajoutant lentement goutte à goutte le chlorure d'acide de formule (V) dans le mélange de déshalogénation et en distillant lentement les produits de la réaction.

6. 1,1-difluoro-2-halogénoéthyl-(halogéno)méthylcétones de formule (IIIa)
Hal'CH₂-CF₂-CO-CH₃₋ₙHalₙ (IIIa),
dans laquelle
n représente 0, 1, 2 ou 3,
Hal représente le chlore, le brome ou l'iode et
lorsque n = 1, 2 ou 3, Hal' représente le chlore, le brome ou l'iode et
lorsque n = 0, Hal' représente le brome.

7. 1,1-difluoro-2-halogénoéthyl-(halogéno)méthylcétones selon la revendication 6, caractérisées en ce qu'il s'agit de la 1,1-difluoro-2-bromoéthyl-bromométhylcétone, de la 1,1-difluoro-2-bromoéthyl-dibromométhylcétone ou de la 1,1-difluoro-2-bromoéthyl-méthylcétone.
